Europäisches Patentamt

European Patent Office

Office européen des brevets

(19)

(11) Veröffentlichungsnummer: **0 065 246**
**B1**

(12) **EUROPÄISCHE PATENTSCHRIFT**

(45) Veröffentlichungstag der Patentschrift:
21.08.85

(21) Anmeldenummer: 82104021.9

(22) Anmeldetag: 08.05.82

(51) Int. Cl.⁴: **C 12 P 1/02**, C 12 N 1/38,
A 61 K 35/72, C 12 N 5/00,
A 23 K 1/16 // C12N1/06,
C12R1/85

(54) **Verfahren zur Gewinnung anaboler, atmungsfördernder, niedermolekularer Wirkstoffe für prophylaktische, therapeutische, zell- und gewebekulturtechnische Zwecke.**

(30) Priorität: 14.05.81 CH 3131/81

(43) Veröffentlichungstag der Anmeldung:
24.11.82 Patentblatt 82/47

(45) Bekanntmachung des Hinweises auf die Patenterteilung:
21.08.85 Patentblatt 85/34

(84) Benannte Vertragsstaaten:
AT BE DE FR GB IT NL SE

(56) Entgegenhaltungen:
EP - A - 0 039 415
BE - A - 712 331
FR - A - 994 079
FR - A - 2 186 247

(73) Patentinhaber: ELKAWI AG, Gartenstrasse 7,
CH-6301 Zug (CH)

(72) Erfinder: Jaeger, Karl-Heinz, Matthofstrand 9,
CH-6005 Luzern (CH)

(74) Vertreter: Utiger, Heinrich, Prof. Dr. phil., David
Hessweg 16 Postfach 145, CH-8060 Zürich (CH)

## Beschreibung

Die vorliegende Erfindung betrifft ein Verfahren zur Gewinnung anaboler, atmungsfördernder, niedermolekularer Wirkstoffe für prophylaktische, therapeutische, zell- und gewebekulturtechnische Zwecke.

Es ist bekannt, daß Hefepilze aus der Familie der Saccharomycetes einen hohen Gehalt an Wirkstoffen, wie Vitamine, Fermente und Spurenelemente enthalten. Besonders seien die Riboflavine und ihre Derivate, die Vitamine des B-Komplexes, oder die Wirkstoffe der Atemkette, wie der Cytochrom C erwähnt, die für Mensch und Tier als äußerst wertvoll zu bezeichnen sind.

Außer diesen bekannten, wachstumsfördernden Stoffe enthalten Hefepilze aber auch noch zum Teil unbekannte Wachstumsfaktoren, die als echte Anabolica bezeichnet werden können, wie sie etwa in embryonalen Geweben, insbesondere auch im Kälberblut enthalten sind, ohne die ein Wachstum von Zellen in vitro nicht stattfinden würde.

Interessanterweise konnte festgestellt werden, daß solche chemisch noch unbekannte, wachstums- und atmungsfördernde Faktoren aus Hefepilzen mit Wirkstoffen aus Kälberserum funktionell eine große Ähnlichkeit besitzen, die sich in der Erhöhung der Resistenz von Mensch und Tier gegen bakterielle, virale und andere Schädigungen positiv ausdrückt.

Gemäß der Patentschrift FR-A-994 079 ist ein Verfahren zur Erhaltung von Hefeextrakten bekannt, bei dem die Plasmolyse der Hefezellen mittelst Ultraschalleinwirkung erfolgt, wobei die nachfolgende Autolyse die Eiweißstoffe in lösliche Stickstoffverbindungen zerlegt und wobei das erhaltene Gemisch von Polypeptiden, Peptide und Aminosäuren als Nährextrakt Verwendung findet. Ein Kombinationsabbau durch Ultraschall unter gleichzeitiger sehr beschränkter Einwirkung von einem proteolytischen Ferment wird erwähnt.

Durch die Patentschrift FR-A-2 186 247 wird ein Fabrikationsverfahren, sowie eine Medikamentenpräparation lyophilisierter Hefezellen beschrieben. Die Hefezellen von Saccharomyces cerevisiae werden vor dem Lyophilisationsvorgang an einen Hefewachstumsfaktor gebunden, der aus einem Hydrolysat, einem Autolysat, einem Proteolysat oder Bier stammt. Der unbekannte Wachstumsfaktor ist nicht näher definiert. Der Begriff des Wachstumsfaktors ist zudem derart weit gefaßt, weil sich dieser nicht näher präzisieren ließ.

Aus dem Stande dieser Technik konnte nichts abgeleitet werden um die Wirkstoffe gemäß vorliegender Erfindung vorwegzunehmen.

Es wurde nun versucht, in Hefeplasmolysaten derartige therapeutisch anwendbare Stoffe aufzufinden, insbesondere auch solche mit regenerierender Wirkung auf Abbauprozeß.

In der Folge zeigte es sich aber, daß es nicht ohne weiteres möglich war, Stoffe mit regenerierender Wirkung in einem Hefeplasmolysat nachzuweisen oder daraus zu isolieren, da in einem Hefeplasmolysat eine Vielzahl von Substanzen mit verschiedenster Wirkung enthalten sind.

Regenerative Prozesse benötigen bekanntlich eine hohe Stoffwechselleistung, die mit einer Erhöhung der inneren Atmung verknüpft ist. Damit zum Nachweis der gesuchten Stoffe mit regenerierenden und atmungsfördernden Eigenschaften aus Hefeplasmolysaten, sowie zur Ermittlung der geeignetsten, zu dem Wirkungsprinzip führenden Herstellungsverfahren herangekommen werden kann, mußten daher bestimmte Testverfahren herangezogen werden, mit deren Hilfe das Vorhandensein, sowie die Konzentration dieser Stoffe nachgewiesen werden konnte. Besonders haben sich hierfür Methoden als günstig erwiesen, wie sie bekannterweise für Gewebekulturen in vitro angewendet werden.

Bis anhin war es nicht gelungen, die gesuchten Wirkstoffe aus Hefezellen in chemisch reiner Form darzustellen, da solche nur in geringsten Konzentrationen in der Hefezelle selbst vorliegen.

Überraschenderweise konnte festgestellt werden, daß aus einem Hefeplasmolysat niedermolekulare Wirkstoffe mit atmungssteigernden, resp. stoffwechselaktivierenden und regenerationsfördernden Eigenschaften gewonnen werden können, wenn man das erfindungsgemäße Verfahren zu ihrer Herstellung anwendet.

In der genuinen Hefezelle sind diese Wirkstoffe nicht oder nicht ohne weiteres nachweisbar, da sie in gebundener oder inaktiver Form vorliegen und somit dem direkten Nachweis entzogen sind, so daß sie auch therapeutisch nicht in Reaktion treten können. Nach Feststellung solcher Wirkstoffe bestand nun die Aufgabe darin, ein Verfahren zu entwickeln, das gestattet, diese Wirkstoffe aus Hefezellen von den höher molekularen Zellbestandteilen abzutrennen, anzureichern und in einer therapeutisch verwertbaren Form darzustellen.

Versuche haben gezeigt, daß die üblichen Verfahren wie Ausfrieren, Aussalzen, Erhitzen, saure oder alkalische Plasmolyse, Zerreibung usw. der Hefezellen zu keinen befriedigenden Ergebnissen führten. Das Problem, die Hefezellmembran möglichst gründlich und ausreichend zu zerstören, um eine hohe Ausbeute der Wirkstoffe zu erhalten, konnte bis dahin nicht in befriedigender Weise gelöst werden.

In bezug auf die Reinigung der gewonnenen, wachstumfördernden Inhaltsstoffe, war bis anhin keine hierfür geeignete Methode bekannt; insbesondere die Art der Enteiweißung durch Ausfällen der genuienen Eiweiße des Hefezellsaftes mittels der üblichen Methoden, wie z. B. Erhitzen oder durch Anwendung von Trichloressigsäure, war nicht befriedigend, da ein solches Ausfällen des Eiweißes auch die gesuchten Substanzen mitausfällte.

Es wurde nun gefunden, daß der Aufschluß der Hefezellen am wirkungsvollsten dann erfolgt, wenn man einer Hefemasse eine zuckerhaltige Lösung, wie Melasse, Fruchtsaftabfälle und dergleichen zusetzt, diese Hefemasse während 1 bis 3 Tagen bei 25°C ± 3°C unter guter Belüftung einem Gärprozeß überläßt und die so erhaltene Hefemassensuspension unter Zusatz eines eiweißspaltenden Fermentes, wie z. B. Papainum, beim Optimum der Gärung gleichzeitig mittels eines Ultraschallintegrators von 50 Watt/cm² Leistung solange wirbelt, bis die Temperatur der Hefesuspension auf ca. 40°C gestiegen ist, was bei einem Überstand von 70—75% der Fall ist. Die abgekühlte, auf ein pH von 6,2 abgepufferte Lösung wird hierauf portionenweise abzentrifugiert. Die Überstände der Zentrifugate werden vereinigt und alsdann einer Schlußdialyse unterworfen, die gegen neutral gepuffertes Wasser erfolgt. Daraufhin wird das Ganze in schonendster Weise eingeengt. Um das erhaltene Produkt zu konservieren, wird diesem ein bekanntes Konservierungsmittel beigegeben.

Überraschenderweise konnte beim erfindungsgemäßen Verfahren festgestellt werden, daß die Ausbeute an atmungsfördernden Wirkstoffen dreimal so groß ist, als bei einer normal angesetzten Papainproteolyse, die ohne gleichzeitige Ultraschalleinwirkung vor sich ging. Dies ist umso überraschender, als dies nicht vorausgesehen werden konnte.

Zum besseren Verständnis des erfindungsgemäßen Verfahrens soll nachfolgend ein Ausführungsbeispiel die Erfindung erläutern.

Ausführungsbeispiel

Man versetze 1 kg Hefepilze, vorzugsweise eine ober- oder untergärige Heferasse der Art Saccharomyces cerevisiae mit 100 Liter Wasser, in dem man ca. 5 kg zuckerhaltige Rohstoffe zusetzt. Das Ganze wird während 1 bis 3 Tagen bei einer Temperatur von ca. 25°C unter guter Belüftung sich selbst überlassen. Hierauf versetze man das Ganze mit 2—5% in bezug auf die Gesamtbiomasse mit einem eiweißspaltenden Ferment, vorzugsweise mit Papainum. Gleichzeitig wird die Gesamtmasse mittels eines Ultraschallintegrators mit 50 Watt/cm² solange gewirbelt, bis die Temperatur der Biomasse auf 40°C gestiegen ist; der Überstand beträgt dann 70—75%. Die Gesamtlösung wird alsdann auf einen pH-Wert von 6,2 gebracht und portionsweise zentrifugiert. Die Zentrifugate werden gesamthaft einer Schlußdialyse unterworfen, die bei ca. 25—30°C gegen neutral gepuffertes Wasser erfolgt. Vor Einleitung der Schlußdialyse werden den Zentrifugaten so viel eines Konservierungsmittels zugesetzt, daß die Endkonzentration desselben am Schluß der Dialyse, im Dialysat bei 0,001—0,008% liegt. Als Konservierungsmittel kann in vorteilhafterweise der p-amino-Benzoesäuremethyl- und/oder äthylester verwendet werden.

Es hat sich gezeigt, daß das erwähnte Konservierungsmittel die Sauerstoffaufnahme z. B. eines Leberhomogenates oder einer Hefekultur durch den Wirkstoff höchstens um 10% gegenüber dem Wirkstoff ohne Konservierungsmittelzusatz senkt. Das Schlußdialysat wird alsdann in schonendster Art und Weise auf 50 mg pro ml Dialysat eingeengt.

Die Standardisierung und Testung des Endproduktes erfolgt dadurch, daß man einer üblichen Gewebekultur in vitro das Endprodukt in einer Verdünnung von 1 : 100 bis 1 : 10 000 zusetzt und daß man zu gegebener Zeit eine Zellzählung durchführt. Gegenüber einer Gewebekontrollkultur ohne Wirkstoffzusatz hat es sich gezeigt, daß die Zellenzahl um mindestens 150% ansteigt.

Es ist auch möglich, eine Standardisierung durch direktes Messen der Zellteilungsbeschleunigung von in vitro gezüchteten Zellen wie Fibroplasten menschlicher oder tierischer Herkunft vorzunehmen. Die erhöhte Resistenz wird durch Verabfolgung der erfindungsgemäß gewonnenen Wirkstoffe so bestimmt, daß menschliche oder tierische Zellen mittels toxischen Metallsalzen, wie Blei- oder Quecksilberverbindungen versetzt werden, derart, daß sie in ihrer Teilungsfähigkeit eingeschränkt oder blockiert werden. Der Grad der Deblockierung durch das Einwirken der Wachstumsfaktoren ergibt dann den Grad der Resistenzfähigkeit.

Weitere Untersuchungen haben gezeigt, daß das Endprodukt durch Anregung des Stoffwechsels ganz generell die Sauerstoffaufnahme und zugleich die Mehrproduktion von Adenosintriphosphat fördert. Das Endprodukt katalysiert und aktiviert die Atemkette an dem Niveau der Cytochromoxidase. Aus diesen Gründen erfolgt z. B. im Falle einer Wunde, unter dem Einfluß des Wirkstoffes, eine vermehrte Zellteilung und somit eine raschere Epithelisierung und Abheilung. Bei Messungen der Futterverwertung von Nutztieren hat es sich herausgestellt, daß ein Zusatz des gefundenen Wirkstoffes eine bessere Ausnutzung des Grundfutters bewirkt, so daß solche Tiere ihren erwünschten Reifegrad auch viel schneller erreichen, als Tiere, deren Futter keine Wirkstoffe enthielten.

Auch hat es sich gezeigt, daß z. B. ein Gärvorgang durch Zusatz des erwähnten Wirkstoffes bedeutend aktiviert wird. So konnte festgestellt werden, daß sich der Wirkstoff in der Bierbrauerei mit Erfolg anwenden läßt, indem man einen normalen Gärvorgang der sonst z. B. 8 1/2 Tage dauert, auf 7 Tage reduzieren kann. Der Zeitgewinn von 1 1/2 Tage ist wirtschaftlich gesehen, bedeutend; die Qualität des Bieres ist in beiden Fällen dieselbe.

Die erfindungsgemäß hergestellten Wachstumsfaktoren finden nicht nur therapeutische Verwendung in der Human- und Tiermedizin, sondern auch im technischen Bereich der Gärprozesse, in der Zell- und Gewebezüchtung in vitro und zur besseren Ausnutzung des Grundfutters.

## Patentansprüche

1. Verfahren zur Gewinnung anaboler, atmungsfördernder niedermolekularer Wirkstoffe für prophylaktische, therapeutische, zell- und gewebekulturtechnische Zwecke, dadurch gekennzeichnet, daß Hefepilze der Familie der Saccharomycetes während ihres maximalen Wachstums durch Ultraschalleinwirkung, bei gleichzeitiger Gegenwart proteolytisch wirksamer Fermente aufgeschlossen werden, wobei anschließend der Überstand in bekannter Weise einer Dialyse unterworfen und das Dialysat in bekannter Weise schonendst eingeengt wird.

2. Verfahren nach Patentanspruch 1, dadurch gekennzeichnet, daß als proteolytisch wirksames Ferment Papainum verwendet wird.

3. Verwendung der nach Patentanspruch 1 und 2 erzeugten Wirkstoffe für die Gewinnung von Zell- und Gewebekulturen.

4. Verwendung der nach Patentanspruch 1 und 2 erzeugten Wirkstoffe als Zusatz für die Beschleunigung von Gärprozessen.

5. Verwendung der nach Patentanspruch 1 und 2 erzeugten Wirkstoffe als Tierfutterzusatz.

## Claims

1. Procedure for the extraction of anabolic, respiration-promoting, low-molecular active substances for prophylactic, therapeutic, cell-culture and tissu-culture purposes, characterized by the desintegration of yeast fungi of the Saccharomycetes family at the moment of their maximum growth by means of ultrasound, in the presence of proteolytically active enzymes, the residue then being subjected to dialysis concentrated under extremely careful treatement.

2. Procedure according to claim 1, charakterized by the use of papain as a proteolytic enzyme.

3. Use of the active substances produced according to claim 1 and 2 for the preparation of cell cultures and tissue cultures.

4. Use of the active substances produced according to patent claims 1 and 2 as additive for the acceleration of fermentation processes.

5. Use of the active substances produced according to patent claims 1 and 2 as animal-feed additives.

## Revendications

1. Procédé de production de substances actives anaboliques favorisant la respiration et de faible poids moléculaire pour des fins prophylactiques, thérapeutiques et pour des techniques de culture de cellules et de tissus, caractérisé par le fait, qu'on soumet des levures de la famille des Saccharomycètes au sommet de leur croissance à une action d'ultra-sons en présence de ferments protéolytiques pour réaliser une désintégration, suivit d'une dialyse de la masse biologique dont le dialysat est concentré prudemment.

2. Procédé suivant la revendication 1, caractérisé par le fait, que le ferment éfficace est la papaine.

3. Produits obtenus suivant les revendication 1 et 2 servant pour l'obtention de cultures cellulaires et de tissus.

4. Produits obtenus suivant les revendications 1 et 2 servant comme additivs accélérants des procédés de fermentations.

5. Produits obtenus suivant les revendications 1 et 2 servant comme additivs pour le fourrage.